(19) Europäisches Patentamt
European Patent Office
Office européen des brevets

(11) **EP 0 747 737 A1**

(12) **EUROPEAN PATENT APPLICATION**

(43) Date of publication:
**11.12.1996 Bulletin 1996/50**

(51) Int. Cl.$^6$: **G02B 6/06**, A61B 5/117,
G06K 9/00

(21) Application number: **96108937.2**

(22) Date of filing: **04.06.1996**

(84) Designated Contracting States:
**DE FR**

(30) Priority: **05.06.1995 JP 137758/95**

(71) Applicant: **HAMAMATSU PHOTONICS K.K.**
**Shizuoka-ken (JP)**

(72) Inventors:
• **Sugawara, Takeo**
**Hamamatsu-shi, Shizuoka-ken (JP)**

• **Nagai, Tsutomu**
**Hamamatsu-shi, Shizuoka-ken (JP)**
• **Hino, Toshihiko**
**Hamamatsu-shi, Shizuoka-ken (JP)**

(74) Representative: **Grünecker, Kinkeldey,**
**Stockmair & Schwanhäusser**
**Anwaltssozietät**
**Maximilianstrasse 58**
**80538 München (DE)**

(54) **Curved fiber optical plate**

(57)     The present invention provides a fiber optical plate which is unitedly formed by a plurality of optical fibers bundled together and comprises (i) an input end surface (2) which is inclined with respect to the center axis of the plurality of optical fibers by an angle at which incident light from within air is prevented from being totally reflected by an interface between a core portion and a clad portion of the plurality of optical fibers; (ii) an output end surface (33) which outputs, of light incident on the input end surface from an object (e.g. a fingerprint) in contact with the input end surface, a light component arriving there after being transmitted through the plurality of optical fibers; and (iii) a curved section (20a) for adjusting the positional relationship between the input end surface and the output end surface, which is formed as the center axis of the optical fibers is curved like an arc. Accordingly, the output end surface is positioned close to the input end surface along the normal direction of the input end surface.

Fig. 1

EP 0 747 737 A1

**Description**

BACKGROUND OF THE INVENTION

Field of the Invention

The present invention relates to a fiber optical plate unitedly formed as a plurality of optical fibers are bundled together.

Related Background Art

Conventionally, as described in United States Patent No. 4,932,776, for example, a fiber optical plate (FOP) in which numerous optical fibers are bundled and united together has been known. At one end of this FOP, an input end face which has been obliquely cut with respect to the optical axis (center axis) is formed. When a finger of a person or the like comes into close contact with this input end face, an irregularity pattern of the surface area of the finger in contact with this input end surface is projected onto an output end surface opposite thereto. As a result, a pattern image of a fingerprint can be read out by CCD (Charge Coupled Device), for example.

SUMMARY OF THE INVENTION

An object of the present invention is to provide a fiber optical plate having an output end surface disposed close to an input end surface in the normal direction of the input end surface.

Another object of the present invention is to provide a fiber optical plate which, when loaded in an apparatus such as a touch sensor or a fingerprint reader, a touch surface at which its input end surface is exposed flush can be made flat, whereby the whole apparatus can be made thin.

In order to attain the above-mentioned objects, the present invention provides a fiber optical plate which is unitedly formed by a plurality of optical fibers bundled together and comprises (i) an input end surface which is inclined with respect to the center axis of the plurality of optical fibers by an angle at which incident light from within air is prevented from being totally reflected by an interface between a core portion and a clad portion of the plurality of optical fibers; (ii) an output end surface which outputs, of light incident on the input end surface from an object in contact with the input end surface, a light component arriving there after being transmitted through the plurality of optical fibers; and (iii) a curved section for adjusting the positional relationship between the input end surface and the output end surface, which is formed as the center axis of the optical fibers is curved like an arc.

In such a fiber optical plate, when the angle of inclination of the input end surface is set to the condition mentioned above, the light which has entered the core portion of the optical fiber from the input end surface after being propagated through the air is gradually attenuated as being propagated through the core, such that it is hardly output from the output end surface. Accordingly, unnecessary light such as background light is hardly output from the output end surface.

On the other hand, when a surface of an object is in close contact with the input end surface, light transmitted through the contact area of the input end surface which is in close contact with this object enters the core portion of the optical fiber. Thus entered light includes a light component which is incident on the interface between the core portion and the clad portion at an angle which satisfies the total reflection condition. Therefore, only the incident light component from the contact area is output from the output end surface.

Also, since a part of the plate has a curved section at which this plate is curved like an arc, the position of the output end surface with respect to the input end surface can be changed. Accordingly, the thickness of the whole plate can be reduced in the normal direction of the input end surface.

Here, preferably, the curved portion renders a curvature, which corresponds to the angle of inclination of the input end surface, to the center axis of the plurality of optical fibers such that the input end surface and the output end surface are positioned substantially orthogonal to each other.

Also, preferably, the angle of inclination of the input end surface is set such that a light-receiving angle 0° is generated for the plurality of optical fibers based on the critical angle of reflection at which the light incident on the clad portion from the core portion within the plurality of optical fibers is subjected to total reflection and the critical angle of refraction generated by the light incident on the core portion of the plurality of optical fibers from within the air at an incident angle of 90°.

Preferably, the plurality of optical fibers include an optical absorber which covers the outer periphery of the clad portion and is made of a material for absorbing the light leaking from the clad portion.

Also, preferably, the plurality of optical fibers are formed by a plurality of units successively fixed along the light advancing direction, while the plurality of units include a slant unit constituting the input end surface and a curved unit which is optically coupled to the slant unit so as to constitute the curved section.

Preferably, the plurality of optical fibers are formed by a plurality of channels arranged and fixed along a direction perpendicular to the light advancing direction, while the plurality of channels respectively have input end surfaces which are arranged so as to shift in parallel to each other.

Also, preferably, the fiber optical plate of the present invention further comprises a contracted section which is formed between the curved section and the output end surface and has a diameter which becomes smaller from the curved portion toward the output end surface.

In this case, desirably, the plurality of optical fibers are formed by a plurality of units successively fixed along the light advancing direction, while the plurality of units include a slant unit constituting the input end surface, a curved unit which is optically coupled to the slant unit so as to constitute the curved section, and a tapered unit which is optically coupled to the curved unit so as to constitute the contracted section.

The present invention will be more fully understood from the detailed description given hereinbelow and the accompanying drawings, which are given by way of illustration only and are not to be considered as limiting the present invention.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will be apparent to those skilled in the art from this detailed description.

BRIEF DESCRIPTION OF THE DRAWINGS

Fig. 1 is a perspective view showing a configuration of a fiber optical plate in accordance with a first embodiment of the present invention;

Fig. 2 is a side view showing the configuration of the fiber optical plate of Fig. 1 in detail;

Fig. 3 is an enlarged plan view showing an end surface of the optical fiber section arranged along the input and output end surfaces of the fiber optical plate of Fig. 1;

Fig. 4 is a table showing each material composition of the core, clad, and optical absorber constituting the optical fiber section of Fig. 3 in % by weight, while indicating the respective refractive indexes of these core and clad;

Fig. 5 is an enlarged cross-sectional view showing a partial configuration of the fiber optical plate of Fig. 1 near its input end surface along the light advancing direction;

Fig. 6 is an enlarged vertical cross-sectional view showing a partial configuration of the fiber optical plate of Fig. 1 near its input end surface along the light advancing direction in a state where the surface of a finger is in close contact with the input end surface thereof;

Fig. 7 is a cross-sectional view showing a state where the input end surface of the fiber optical plate of Fig. 1 is disposed at a position identical to that of a touch surface of a touch sensor, a photosensor section of a fingerprint reader, or the like;

Fig. 8 is a side view showing a configuration of a fiber optical plate in accordance with a second embodiment of the present invention in detail;

Fig. 9 is an enlarged plan view showing an end surface of the optical fiber section arranged along the input and output end surfaces of the fiber optical plate of Fig. 8; and

Fig. 10 is a side view showing a configuration of a fiber optical plate in accordance with a third embodiment of the present invention in detail.

DETAILED DESCRIPTION OF THE PREFERRED EMBODIMENTS

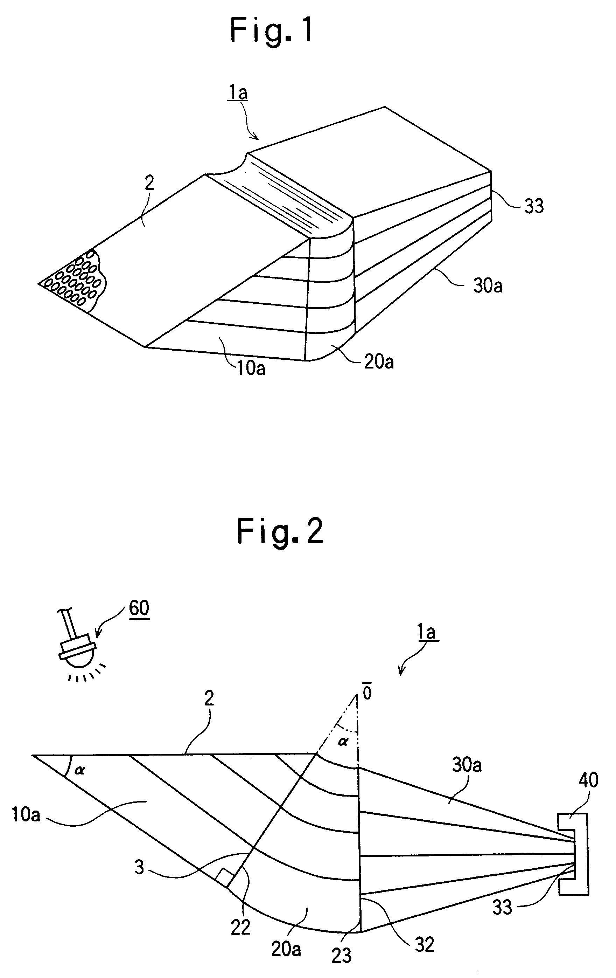

In the following, configurations and operations of various embodiments concerning the fiber optical plate of the present invention will be explained in detail with reference to Figs. 1 to 10. Here, in the explanation of the drawings, constituents identical to each other will be referred to with marks identical to each other without repeating their overlapping explanations. Also, ratios of sizes in the drawings do not always correspond to those in the explanations.

First Embodiment

As shown in Figs. 1 and 2, a fiber optical plate (FOP) 1a of this embodiment is used as part of a photosensor section for a fingerprint reader. This FOP 1a is constituted by a plurality of units optically coupled to each other in succession along the light advancing direction, such that a slant FOP (slant unit) 10a, a curved FOP (curved unit) 20a, and a tapered FOP (tapered unit) 30a are unitedly formed together with their respective end faces being joined to each other. Namely, an output end surface 3 of the slant FOP 10a and an input end surface 22 of the curved FOP 20a are bonded and fixed together by means of a light-transmitting adhesive. An output end surface 23 of the curved FOP 20a and an input end surface 32 of the tapered FOP 30a are bonded and fixed together by means of a light-transmitting adhesive. Here, for convenience, the directions of fibers within various FOPs are indicated by continuous lines in a side surface of the FOP 1a.

The slant FOP 10a is unitedly formed by a plurality of optical fibers bundled together and shaped like a triangle pole as a whole. In this slant FOP 10a, the output end surface 3 has been cut perpendicular to the optical axis (center axis) of the optical fibers, while its input end surface 2 has been cut with respect to the optical axis (center axis) of the optical fibers at a predetermined angle (slant angle) $\alpha$ ($0° < \alpha < 90°$). This slant angle $\alpha$ of the input end surface 2 will be explained in detail later.

The curved FOP 20a is unitedly formed as a fiber plate in which a plurality of optical fibers are bundled together is curved like an arc. In this curved FOP 20a, the input end surface 22 is bonded to the output end surface 3 of the slant FOP 10a, while the angle of the input end surface 22 and the output end surface 23 with respect to each other is set to an angle $\alpha$ which is identical to the slant angle $\alpha$ of the input end surface 2 of the slant FOP 10a. Accordingly, the output end surface 23 of the curved FOP 20a is positioned orthogonal to the input end surface 2 of the slant FOP 10a.

The tapered FOP 30a is unitedly formed by a plurality of optical fibers bundled together and has a taper form in which its diameter continuously decreases from the input end surface 32 toward its output end surface 33. The input end surface 32 of this tapered FOP 30a is bonded to the output end surface 23 of the curved FOP 20a. Due to this configuration, the output end surface 33 of the curved FOP 30a is positioned orthogonal to the input end surface 2 of the slant FOP 10a.

Consequently, an optical image input from the curved FOP 20a to the tapered FOP 30a reduces its size as being propagated through the tapered FOP 30a, whereby thus reduced image (object image) is obtained from the output end surface 33 of the tapered FOP 30a. Accordingly, when a light source 60 is disposed in front of the input end surface 2 of the slant FOP 10a while a CCD 40 is attached to the output end surface 33 of the tapered FOP 30a, the reduced image can be detected. Thus configured image detecting unit can be formed compact in a thin shape along the normal direction of the input end surface 2 of the slant FOP 10a.

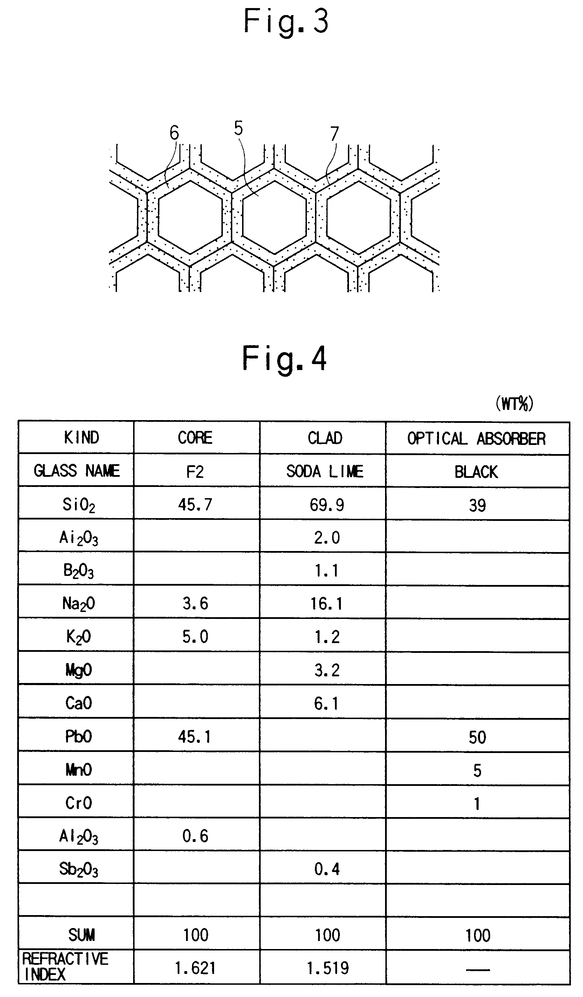

As shown in Fig. 3 under magnification, each of these various FOPs 10a, 20a, and 30a is constituted by numerous optical fibers bundled together in close contact with each other, while each optical fiber comprises a core 5, a clad 6 covering the outer peripheral portion of this core 5, and an optical absorber 7 covering the outer peripheral portion of the clad 6. Since the optical absorber 7 is disposed at this position, the light leaking from the core 5 and the light incident on the clad 6 are absorbed, whereby the neighboring optical fibers are optically insulated from each other by this optical absorber 7.

As shown in Fig. 4, the cores 5, clads 6, and optical absorbers 7 in the FOPs 10a, 20a, and 30a are made of respectively common materials. Namely, each core 5 is an F2 glass made of $SiO_2$, $PbO$, $K_2O$, $Na_2O$, and $Al_2O_3$ and has a refractive index of 1.621. Each clad 6 is a soda lime glass made of $SiO_2$, $Na_2O$, $CaO$, $MgO$, $Al_2O_3$, $K_2O$, $B_2O_3$, and $Sb_2O_3$ and has a refractive index of 1.519. Each optical absorber 7 is a black glass made of $PbO$, $SiO_2$, $MnO$, and $CrO$.

As each of the FOPs 10a, 20a, and 30a is formed by optical fibers each of which has the optical absorber 7 covering the outer peripheral portion of the clad 6, the light which has entered the core 5 is prevented from leaking therefrom and entering the neighboring core 5. Accordingly, crosstalk can be prevented from occurring between the neighboring fibers, thereby improving the S/N ratio of the optical image output from the tapered FOP 30a. This effect is independent of the slant angle $\alpha$ of the input end surface 2 of the slant FOP 10a. Namely, no matter how acute the slant angle $\alpha$ of the input end surface 2 is, the light which has entered the core 5 of the input end surface 2 is propagated through the corresponding cores 5 of the FOPs 10a, 20a, and 30a so as to be output from the output end surface 33.

Here, the slant angle $\alpha$ of the input end surface 2 in the slant FOP 10a will be explained.

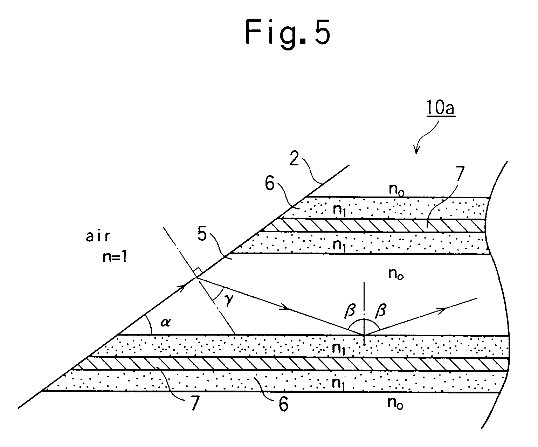

Fig. 5 shows a vertical cross section of the slant FOP 10a. In this drawing, $n_0$, $n_1$, and $n$ indicate the refractive index of the core 5, the refractive index of the clad 6, and the refractive index of the air surrounding the slant FOP 10a, respectively. In the slant FOP 10a of this embodiment, the slant angle $\alpha$ of the input end surface 2 is defined such that the light-receiving angle becomes 0°. Namely, the slant angle $\alpha$ is set to an angle at which no total reflection occurs at the interface between the core 5 and the clad 6 when light is incident thereon at any angle from within the air.

Assuming that the critical angle of reflection at the interface between the core 5 and the clad 6 is $\beta$ and that the critical angle of refraction at the interface between the air and the core 5 is $\gamma$, maximum value $\alpha_0$ of the slant angle $\alpha$ can be determined by the following equations (1) to (3):

$$n_0\sin\beta = n_1\sin90° \quad \text{[condition for total reflection]} \tag{1}$$

$$n_0\sin\gamma = n\sin90° \quad \text{[condition for light-receiving angle 0°]} \tag{2}$$

$$\alpha + (90°+\gamma) + (90°-\beta) = 180° \tag{3}$$

In this case, since $n_0=1.621$, $n_1=1.519$, and $n=1$, $\alpha_0=31.54°$ is determined. Accordingly, when the slant angle $\alpha$ of the input end surface 2 is set to a value not higher than the $\alpha_0$ value of 31.54°, the light which has been propagated through the air and then is made incident on the input end surface 2 does not generate total reflection at the interface between the core 5 and the clad 6 regardless of its incident angle.

Accordingly, the light which has entered the slant FOP 10a from the input end surface 2 after having been propagated through the air is gradually attenuated as a part thereof leaks from the core 5 to the clad 6 so as to be absorbed by the optical absorber 7 in the process of being repeatedly refracted and propagated through the slant FOP 10a. Thus, when the slant angle $\alpha$ of the input end surface 2 is set to such an angle of 31.54°, the light which has entered the slant FOP 10a from the input end surface 2 after having been propagated through the air is hardly output from the output end surface 3.

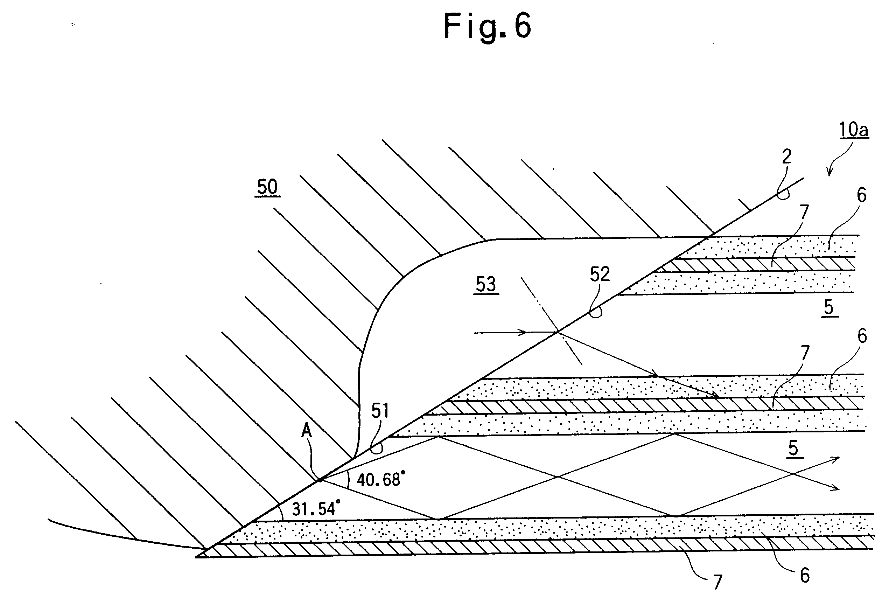

As shown in Fig. 6, when a specific object such as a surface of a finger 50 is in close contact with the input end surface 2 of the slant FOP 10a, a contact area 51, at which a convex portion of the finger 50 is in close contact with the input end surface 2, and a non-contact area 52, at which a concave portion of the finger 50 is separated from the input surface 2 by a space 53, are formed according to irregularities of the finger 50. At the contact area 51, the input end surface 2 and the surface of the finger 50 are in close contact with each other, while the refractive index of the finger surface is greater than that of the air. Accordingly, at the contact area 51, the relationship between the incident angle of the light entering the input end surface 2 and the total reflection condition at the interface between the core 5 and the clad 6 changes from that before the finger 50 comes into contact with the input end surface 2. Consequently, there exists light which is incident on the interface between the core 5 and the clad 6 at an angle which satisfies the total reflection condition.

For example, as shown in Fig 6, it is assumed that light is emitted from behind the finger 50 by a light source 60 or the like and that the light transmitted therethrough has reached a point A within the contact area 51. Of the light entering the slant FOP 10a from this point A, as depicted, a light component having an incident angle within the range of 40.68°, the center of which range coincides with the center axis of the core 5, satisfies the total reflection condition indicated by equation (1) at the interface between the core 5 and the clad 6. This incident light component repeats total reflection at the interface between the core 5 and the clad 6 so as to be output from the output end surface 3, thereby entering the curved FOP 20a. Here, a light component with an incident angle outside of the above-mentioned range is attenuated and extinguished in the process of being propagated with repeated refraction.

At the non-contact area 52, by contrast, the space 53, in which the air exists, is formed between the input end surface 2 and the finger 50. Namely, the state thereof is not changed at all from that before the finger 50 comes into contact therewith. Accordingly, though light enters the core 5 from the non-contact area 52 of the input end surface 2, it still does not satisfy the total reflection condition at the interface between the core 5 and the clad 6 regardless of its incident angle and thereby is attenuated and extinguished in the process of being propagated through the core 5.

Accordingly, when a surface image of an object or the like is to be detected, in the slant FOP 10a, only incident light from the convex portions in the object surface which are in close contact with the input end surface 2 is output from the output end surface 3, whereas incident light from the concave portions in the object surface is attenuated and extinguished in the process of being propagated through the FOPs 10a, 20a, and 30a. Accordingly, while unnecessary light such as background light is eliminated, only the image of the part of the object surface which is in close contact with the input end surface 2 is transmitted and output.

While the example explained above relates to a case where the slant angle $\alpha$ of the input end surface 2 is set to 31.54° with respect to N.A. (numerical aperture) of 0.55, the slant angles $\alpha$ with respect to other typical N.A. values are shown in Table 1.

Table 1

| N.A. | Slant angle $\alpha$ |
|---|---|
| 0.35 | 36.96° |
| 0.55 | 31.54° |
| 0.88 | 25.20° |
| 1.00 | 21.89° |

In the FOP 1a of this embodiment, since the slant FOP 10a disposed at a position which becomes the input section for the front stage has such a characteristic, when fingertips and the like are not in contact with the input end surface 2, light such as background light is not output at all from the output end surface 33 of the tapered FOP 30a which functions as the final stage. Therefore, when a fingertip is in contact with the input end surface 2, an image of its fingerprint is output from the output end surface 33.

Since the surface image of the finger in contact with the input end surface 2 is output from the output end surface 3 with a high contrast, the FOP 1a is suitably used as a sensor section for a fingerprint reader. In addition, since light is output from the output end surface 33 only when a finger or the like is in contact with the input end surface 2, the FOP 1a can also be used as a normal touch sensor for detecting whether a light-transmitting object is in contact with the input end surface 2 or not.

Also, by means of the curved FOP 20a, the object image of the input end surface 2 in the slant FOP 10a is quite efficiently transferred to the output end surface 33 of the tapered FOP 30a, whereby information about the object image can be obtained with an excellent S/N at the CCD 40.

Here, assuming that the reducing ratio of N.A. (numerical aperture) in the tapered FOP 30a is r:1, there is a relationship expressed by the following equation (4):

$$NA(L)=(1/r)\times NA(S) \tag{4}$$

wherein NA(L) and NA(S) indicate the numerical apertures of the input end surface 32 and output end surface 33 of the tapered FOP 30a, respectively.

For example, when the reducing ratio of N.A. is 5:1 at NA(S)=1.0, NA(L) becomes 0.2. In this case, the light-receiving angle at the input end surface 32 of the tapered FOP 30a becomes very small, namely, 11.5°. In other words, in such a case where the reducing ratio is large, it is necessary for the object image to be incident on the input end surface 32 of the tapered FOP 30a as perpendicularly as possible. Since the object image is input into the input end surface 32 of the tapered FOP 30a with nearly a right angle by means of the curved FOP 20a, it is efficiently transmitted to the output end surface 33 of the tapered FOP 30a. Here, when the curved FOP 20a which has been curved like an arc around a central axis O (cf. Fig. 2) is cut at a plane including the central axis O, thus cut surface becomes perpendicular to the optical axes of optical fibers constituting the curved FOP 20a.

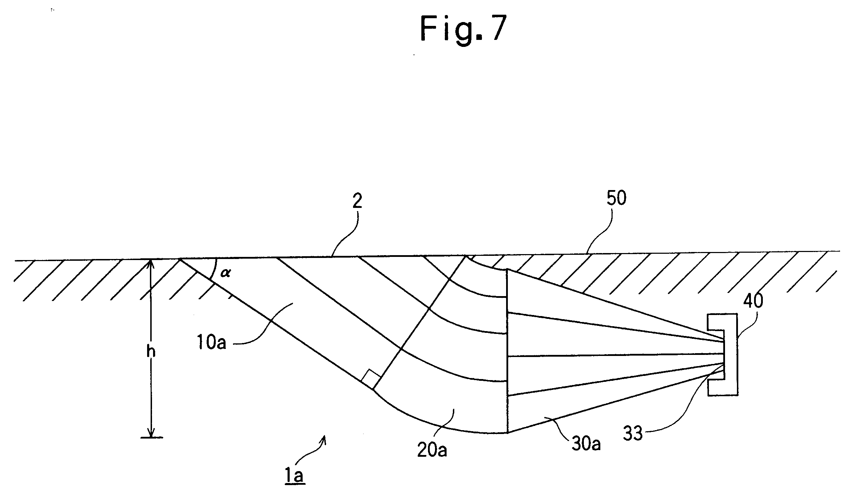

Since the input end surface 2 and the output end surface 33 can be disposed orthogonal to each other as shown in Fig. 7, when the FOP 1a is used in a touch sensor, a photosensor section of a fingerprint reader, or the like such that the input end surface 2 is disposed at the surface position identical to that of the touch surface 50, thereby making the touch surface 50 flat. In this case, the CCD 40, other devices, and the like can be disposed behind the output end surface 33 along the touch surface 50. Accordingly, the whole image detection unit can be accommodated within a thickness h of the FOP 1a in the normal direction of the input end surface 2, thereby making it possible for a thin apparatus to sufficiently incorporate this unit therein.

Second Embodiment

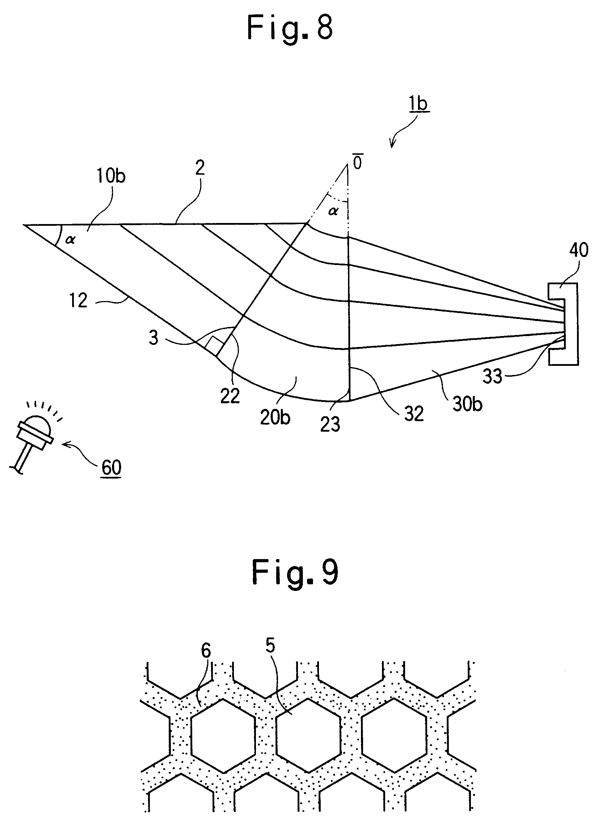

An FOP 1b of this embodiment shown in Fig. 8 is also used as a touch sensor, a photosensor of a fingerprint reader, or the like. A curved FOP 20b and a tapered FOP 30b constituting this FOP 1b are formed similar to the curved FOP 20a and the tapered FOP 30a in the above-mentioned first embodiment, respectively. However, its slant FOP 10b, unlike the slant FOP 10a in the first embodiment, is unitedly formed by a plurality of normal optical fibers bundled together, each of which comprises the clad 6 covering the outer periphery of the core 5 as shown in Fig. 9 without using the optical absorber 7.

When such a type of FOP is used, the illumination light source 60 can be disposed so as to face a bottom surface 12 of the slant FOP 10b. When the light source 60 is disposed at this position, illumination light emitted from the light source 60 enters the slant FOP 10b from the bottom surface 12 and is transmitted through each of the bundled optical fibers to reach the input end surface 2, thereby illuminating it from the bottom surface side. When a finger or the like is in contact with the input end surface 2, its contact area is irradiated with the irradiation light and only light reflected thereby is propagated through the optical fiber by total reflection therewithin. Then, as in the case of the above-mentioned first embodiment, the reflected light which has entered the tapered FOP 30b by way of the slant FOP 10b and the curved FOP 20b is output from the output end surface 33.

Here, also in order to obtain an object image with a favorable S/N from the output end surface 33, the direction of illumination by the light source 60 is desirably set such that the direction of reflection at the input end surface 2 substantially coincides with the optical axis direction of the optical fibers constituting the FOP. Also, the curved FOP 20b and the tapered FOP 30b may be constituted by such FOPs having no optical absorber 7. It can be arbitrarily selected which type of FOP is used therefor.

Third Embodiment

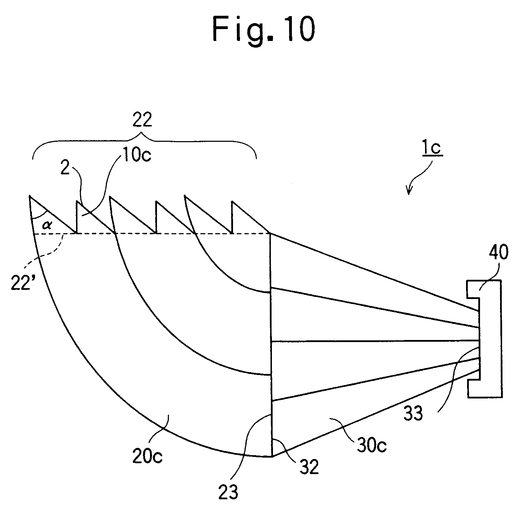

As shown in Fig. 10, an FOP 1c of this embodiment is also used as a touch sensor, a photosensor of a fingerprint reader, or the like. Unlike the above-mentioned first and second embodiments, this FOP 1c is formed as a curved FOP 20c, which includes a slant FOP at its surface area, and a tapered FOP 30c, which is configured similar to the tapered FOPs 30a and 30b of the first and second embodiments, are bonded together.

In this case, the input end surface 22 of the curved FOP 20c is cut like sawteeth. It is equivalent to the case where small slant FOPs 10c are continuously arranged in a single row at a virtual flat input end surface 22' of the curved FOP 20c. Namely, this FOP 1c are formed by a plurality of channels arranged and fixed in a direction perpendicular to the light advancing direction, and has the slant FOPs 10c which are shifted channel by channel in parallel to each other.

The input end surface 2 at each of these plurality of slant FOPs 10c has a slant angle which is set to an angle α at which the light which is incident on the input end surface 2 after having been propagated through the air does not satisfy the total reflection condition at the interface between the core 5 and the clad 6. Since the input end surface 22 of the curved FOP 20c is cut like sawteeth in this manner, unnecessary background light is prevented from being output from the output end surface 33 of the tapered FOP 30c.

In the embodiments explained in the foregoing, three kinds of FOPs configured as units, namely, slant, curved, and tapered FOPs, are formed as being bonded together. However, a single FOP having a partial curve, contraction, and the like may be used as the whole of FOPs, of course.

Also, only the cases where the input end surface of the slant FOP and the output end surface of the tapered FOP are orthogonal to each other are exemplified. However, when the slant angles of the input end surfaces and output end surfaces of various FOPs are changed, their positional relationship can also be changed.

As explained in detail in the foregoing, in the fiber optical plate in accordance with the present invention, since a curved section in which this plate is curved like an arc is formed at a part of the plate, when such an FOP is used for constituting a touch sensor, a photosensor of a fingerprint reader, or the like, the output end surface thereof is prevented from protruding below the input end surface, while there is no necessity for cutting the optical fiber into short strips along the light advancing direction. Accordingly, the touch surface can be formed flat, while the whole apparatus can be made thin. Also, since the slant angle of the input end surface is set to an angle at which the light incident on the core from within the air does not generate total reflection at the interface between the clad and the core, when the surface of an object is in close contact with the input end surface, unnecessary light such as background light is prevented from being output from the output end surface, whereby only the image of the part which is in contact with the input end surface can be output.

From the invention thus described, it will be obvious that the invention may be varied in many ways. Such variations are not to be regarded as a departure from the spirit and scope of the invention, and all such modifications as would be obvious to one skilled in the art are intended for inclusion within the scope of the following claims.

The basic Japanese Application No. 137758/1995 (7-137758) filed on June 5, 1995 is hereby incorporated by reference.

## Claims

1. A fiber optical plate which is unitedly formed by a plurality of optical fibers bundled together, said fiber optical plate comprising:

    an input end surface which is inclined with respect to a center axis of said plurality of optical fibers by an angle at which incident light from within air is prevented from being totally reflected by an interface between a core portion and a clad portion of said plurality of optical fibers;
    an output end surface which outputs, of light incident on said input end surface from an object in contact with said input end surface, a light component arriving there after being transmitted through said plurality of optical fibers; and
    a curved section for adjusting a positional relationship between said input end surface and said output end surface, said curved section being formed as a center axis of said optical fibers is curved like an arc.

2. A fiber optical plate according to claim 1, wherein said curved portion renders a curvature, which corresponds to the angle of inclination of said input end surface, to the center axis of said plurality of optical fibers such that said input end surface and said output end surface are positioned substantially orthogonal to each other.

3. A fiber optical plate according to claim 1, wherein the angle of inclination of said input end surface is set such that a light-receiving angle 0° is generated for said plurality of optical fibers based on a critical angle of reflection at which light incident on said clad portion from said core portion within said plurality of optical fibers is subjected to total reflection and a critical angle of refraction generated by light incident on said core portion of said plurality of optical fibers from within the air at an incident angle of 90°.

4. A fiber optical plate according to claim 1, wherein said plurality of optical fibers include an optical absorber which covers an outer periphery of said clad portion and is made of a material for absorbing light leaking from said clad portion.

5. A fiber optical plate according to claim 1, wherein said plurality of optical fibers are formed by a plurality of units successively fixed along a light advancing direction and wherein said plurality of units include a slant unit constituting said input end surface and a curved unit which is optically coupled to said slant unit so as to constitute said curved section.

6. A fiber optical plate according to claim 1, wherein said plurality of optical fibers are formed by a plurality of channels arranged and fixed along a direction perpendicular to a light advancing direction and wherein said plurality of channels respectively have input end surfaces which are arranged so as to shift in parallel to each other.

7. A fiber optical plate according to claim 1, further comprising a contracted section which is formed between said curved section and said output end surface and has a diameter which becomes smaller from said curved portion toward said output end surface.

8. A fiber optical plate according to claim 7, wherein said plurality of optical fibers are formed by a plurality of units successively fixed along a light advancing direction and wherein said plurality of units include a slant unit constituting said input end surface, a curved unit which is optically coupled to said slant unit so as to constitute said curved section, and a tapered unit which is optically coupled to said curved unit so as to constitute said contracted section.

# Fig.1

# Fig.2

# Fig. 3

# Fig. 4

(WT%)

| KIND | CORE | CLAD | OPTICAL ABSORBER |
|---|---|---|---|
| GLASS NAME | F2 | SODA LIME | BLACK |
| $SiO_2$ | 45.7 | 69.9 | 39 |
| $Ai_2O_3$ | | 2.0 | |
| $B_2O_3$ | | 1.1 | |
| $Na_2O$ | 3.6 | 16.1 | |
| $K_2O$ | 5.0 | 1.2 | |
| MgO | | 3.2 | |
| CaO | | 6.1 | |
| PbO | 45.1 | | 50 |
| MnO | | | 5 |
| CrO | | | 1 |
| $Al_2O_3$ | 0.6 | | |
| $Sb_2O_3$ | | 0.4 | |
| | | | |
| SUM | 100 | 100 | 100 |
| REFRACTIVE INDEX | 1.621 | 1.519 | — |

# Fig.5

# Fig.6

EP 0 747 737 A1

# Fig.7

# Fig. 8

# Fig. 9

# Fig. 10

**European Patent Office**

## EUROPEAN SEARCH REPORT

Application Number

EP 96 10 8937

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DE-A-44 04 918 (MITSUBISHI ELECTRIC CORP) 18 August 1994 * column 11, line 26 - column 12, line 48; figures 8-10 * * column 19, line 50 - column 20, line 11; figures 29,30 * | 1-4,7 | G02B6/06 A61B5/117 G06K9/00 |
| A | * figures 2,24 * | 5,8 | |
| A,D | US-A-4 932 776 (DOWLING JR ROBERT F ET AL) 12 June 1990 * column 5, line 27 - column 6, line 38; figure 4 * | 1,6 | |
| A | US-A-3 874 783 (COLE HENRY B) 1 April 1975 * column 5, line 18 - line 57; figures 8,9 * | 8 | |

|  |  |
|---|---|
|  | TECHNICAL FIELDS SEARCHED (Int.Cl.6) |
|  | G02B A61B G06K G07C |

The present search report has been drawn up for all claims

| Place of search | Date of completion of the search | Examiner |
|---|---|---|
| BERLIN | 11 September 1996 | von Moers, F |